# EUROPÄISCHE PATENTSCHRIFT

(11) **EP 2 809 639 B1**
(45) Veröffentlichungstag und Bekanntmachung des Hinweises auf die Patenterteilung: **13.01.2016**
(21) Anmeldenummer: 13700258.0
(22) Anmeldetag: 15.01.2013
(51) Int. Cl.: C07C 41/01, C07C 43/04, C01B 3/34, C01B 3/36, C01B 31/18, C07C 11/04, C07C 11/06, C07C 1/20, C07C 1/22

(54) **DIREKTSYNTHESE VON DME AM GLEICHGEWICHT**
DIRECT SYNTHESIS OF DME IN EQUILIBRIUM
SYNTHÈSE DIRECTE DE DME À L'ÉQUILIBRE

(30) Priorität: 31.01.2012 DE 102012001811; 31.01.2012 DE 102012001803; 21.02.2012 EP 12001135
(43) Veröffentlichungstag der Anmeldung: 10.12.2014
(73) Patentinhaber: Linde AG, 80331 München (DE)
(72) Erfinder: SCHÖDEL, Nicole, 81477 München (DE); HAIDEGGER, Ernst, 85521 Riemerling (DE); SCHMIGALLE, Holger, 82538 Geretsried (DE); GÖKE, Volker, 82538 Geretsried (DE); THALLER, Christian, 80687 München (DE); SCHMADERER, Harald, 82515 Wolfratshausen (DE)
(86) Internationale Anmeldenummer: PCT/EP2013/000101
(87) Internationale Veröffentlichungsnummer: WO 2013/113467

(56) Entgegenhaltungen:
- EP-A2- 2 119 668
- GB-A- 2 097 382
- JP-A- 2000 103 757
- US-A1- 2006 287 405

## Beschreibung

Die Erfindung betrifft ein Verfahren zur Herstellung von DME (Dimethylether).

Ein derartiges Verfahren weist zumindest die Schritte auf: Erzeugen eines Synthesegases enthaltend CO und H₂ in einer Einrichtung zur Synthesegaserzeugung (z.B. in einem Synthesegasreaktor), wobei unter einer solchen Einrichtung jegliche zur Synthesegaserzeugung geeignete Einrichtung verstanden wird, und Einleiten des Synthesegases in einen DME-Reaktor zur Direktsynthese von DME durch katalysierte Umsetzung des Synthesegases mittels eines Katalysators unter Bildung eines Produktstromes (Reaktoreffluent) enthaltend DME, CO₂, H₂O, CH₃OH und nicht umgesetztes Synthesegas (CO und H₂).

Aus der EP2119668 ist ein Verfahren zur Synthesegaserzeugung bekannt. Die GB2097382 beinhaltet ein Verfahren zur Umsetzung von Synthesegas in Dimethylether. Auch US2006/0287405 beinhaltet ein Verfahren zur Dimethyletherherstellung, wobei Kohlendioxid aus dem Produktstrom abgetrennt wird. Auch die JP2000 103757 beinhaltet ein Verfahren zur Herstellung von Dimethylether.

Das Synthesegas aus CO und H₂ kann dabei aus Erdgas hergestellt werden, beispielsweise durch Dampfreformierung:

CH₄+H₂O -> CO + 3H₂.

Daneben besteht die Möglichkeit Erdgas auch durch partielle Oxidation zu Synthesegas umsetzen:

2CH₄ + O₂ -> 2CO + 4H₂.

Ferner kann auch durch die sogenannte autotherme Reformierung (Kombination von Dampfreformierung und partieller Oxidation in einem Apparat) Synthesegas erzeugt werden. Die beiden Verfahren werden so miteinander kombiniert, dass der Vorteil der Oxidation (Bereitstellung von Wärmeenergie) sich mit dem Vorteil der Dampfreformierung (höhere Wasserstoffausbeute) vorteilhaft ergänzt.

Schließlich kann auch durch das sogenannte combined Reforming (Kombination von Dampfreformierung und partieller Oxidation in getrennten Apparaten) Synthesegas hergestellt werden.

Die Direktsynthese von DME erfolgt in bekannter Weise aus Synthesegas nach der folgenden Summengleichung:

3H2 + 3CO -> DME + CO₂

Gemäß der Stöchiometrie ist der Einsatz eines Synthesegases mit einem H₂/CO-Verhältnis von etwa 1:1 präferiert.

Als Mechanismus wird aktuell folgender Reaktionspfad über intermediäres Methanol angenommen:

2H₂ + CO -> CH₃OH

2CH₃OH -> DME + H₂O

H₂O+CO -> CO₂+H₂ (Wassergas-Shift-Reaktion).

Ein derartiges Verfahren ist z.B. aus der US 6,458,856 B1 bekannt.

Hierbei erweist es sich als problematisch, dass das Effluent des DME-Reaktors aufgrund der üblicherweise niedrigen Konversionsrate auch eine große Menge an nicht umgesetztem Synthesegas aufweist. Die Gastrennung gestaltet sich somit aufwändig, da nicht umgesetztes Synthesegas in den DME-Reaktor zurückgeführt werden soll, wohingegen das gebildete CO₂ in der erdgasbasierten Ausführung des eingangs dargestellten Verfahrens in den Synthesegasteil (Synthesegaserzeugung) zurückgeführt werden muss.

Hiervon ausgehend liegt daher der vorliegenden Erfindung die Aufgabe zugrunde, ein Verfahren zu schaffen, dass eine vergleichsweise einfache Gastrennung gestattet.

Dieses Problem wird durch ein Verfahren mit den Merkmalen des Anspruchs 1 gelöst.

Danach ist vorgesehen, dass die Direktsynthese am oder nahe am chemischen Gleichgewicht durchgeführt wird, um die Konzentration des Synthesegases im besagten Produktstrom deutlich zu reduzieren. Hierzu wird bevorzugt ein Cu-basierter Katalysator verwendet, der eine acide Funktionalität für eine entsprechende hohe Aktivität und Selektivität für DME aufweist (bifunktionaler Katalysator). Hierbei begünstigt jene Funktionalität insbesondere die Abspaltung von Wasser gemäß 2CH₃OH -> DME + H₂O.

Die Gleichgewichtskonzentration von Dimethylether liegt vor, wenn sich die Reaktion von Kohlenmonoxid und Wasserstoff zu Dimethylether und Kohlendioxid im chemischen Gleichgewicht befindet. Das chemische Gleichgewicht der Reaktion ist erreicht, wenn die Geschwindigkeit der Hinreaktion (3 H₂ + 3 CO → DME + CO₂) gleich der Geschwindigkeit der Rückreaktion (DME + CO₂ → 3 H₂ + 3 CO) ist.

Durch die Erhöhung der Konversionsrate an bzw. nahe an das chemische Gleichgewicht wird die Rückführung des Synthesegases in den DME-Reaktor hinfällig, so dass das nicht umgesetzte Synthesegas und CO₂ gemeinsam in den Synthesegasteil zurückgeführt werden können.

Aufgrund der Eigenschaften früherer Katalysatorsysteme und verfahrenstechnischer Limitierungen war bislang an einen Umsatz nahe an das chemische Gleichgewicht der direkten DME-Synthese nicht zu denken. Durch den vorliegend bevorzugten Einsatz hochaktiver und selektiver Katalysatoren bzw. Katalysatorsysteme ist jedoch der Betrieb des DME-Reaktors am bzw. nahe am chemischen Gleichgewicht möglich, und zwar bevorzugt bei mindestens 70% Umsatz, bevorzugt mindestens 80% Umsatz, bevorzugter 85% Umsatz, am meisten bevorzugt mindestens 90% Umsatz bezogen auf den CO-Anteil des Synthesegases.

Demgegenüber ist der Umsatz im Stand der Technik signifikant geringer (z.B. Umsatz 50% in Demonstrationsanlage gemäß Präsentation: New Clean Fuel DME, Y. Ohno (JFE Holdings, Inc., Japan), DeWitt Global Methanol&MTBE Conference, Bangkok, March 12-14 2007)

Es ist diesbezüglich weiterhin vorgesehen, dass die Direktsynthese im DME-Reaktor zumindest bis zum Erreichen einer Konzentration des DME im Reaktoreffluent von mindestens 70%, mindestens 80%, mindestens 85% oder mindestens 90% der Gleichgewichtskonzentration von DME durchgeführt wird.

Weiterhin wird der aus dem DME-Reaktor abgezogene Produktstrom enthaltend CO, H₂, CO₂, DME, H₂O und Methanol zum Trennen des restlichen (insbesondere niedrig konzentrierten) Synthesegases sowie CO₂ von einer DME-reichen (flüssigen) Phase gekühlt, so dass CO₂, H₂ und CO z.B. über Kopf aus einer Kolonne als gasförmige Phase und DME als Sumpfprodukt in einer flüssigen Phase abgezogen werden können.

Die gasförmige Phase wird dann in den Synthesegasteil (Synthesegaserzeugung) zurückgeführt.

Abhängig von der Anwendung für das DME (Treibmittel, Lösungsmittel, LPG Beimischung, Treibstoff, Einsatzstoff für Olefinsynthese,...) können ggf. weitere Trennschritte durchgeführt werden, um CO₂, Methanol oder Wasser aus der besagten Flüssigphase abzutrennen.

Gemäß einem weiteren Erfindungsgedanken wird ein Verfahren zur Herstellung von DME vorgeschlagen, aufweisend die Schritte:
- Erzeugen eines Synthesegases enthaltend CO und H₂ in einer Einrichtung zur Synthesegaserzeugung,
- Einleiten des Synthesegases in einen DME-Reaktor zur Direktsynthese von DME durch katalysierte Umsetzung des Synthesegases mittels eines Katalysators unter Bildung eines Produktstromes enthaltend DME, CO₂, H₂O, CH₃OH und nicht umgesetztes Synthesegas,
- wobei der Katalysator dazu ausgebildet ist, insbesondere die Synthese von Methanol zu beschleunigen sowie die Wasserabspaltung gemäß 2CH₃OH->DME+H₂O zu beschleunigen, wobei insbesondere der Katalysator ein Cu-basierter Katalysator mit einer aciden Funktionalität ist, und
- wobei in der Direktsynthese gebildetes Kohlendioxid und/oder nicht umgesetztes Synthesegas zur Synthesegaserzeugung zurückgeleitet wird.

Ferner besteht die Möglichkeit, dass das in den DME-Reaktor einzuleitende Synthesegas durch Trockenreformierung hergestellt wird, wobei Methan und Kohlendioxid zu Kohlenmonoxid und Wasserstoff umgesetzt werden.

Die Trockenreformierung wird dabei vorteilhafterweise unter Anwesenheit eines modifizierten, rußresistenten nickelbasierten Katalysators durchgeführt, wie er auch bei Dampfreformierungen ähnlich zur Anwendung kommt. Zweckmäßigerweise wird die Trockenreformierung bei einer Temperatur zwischen 750°C und 950°C durchgeführt.

Methan im Sinne der Erfindung umfasst auch methanhaltige Gase wie z.B. Erdgas.

Unter Trockenreformierung im Sinne der Erfindung versteht man die Umsetzung von Methan oder Erdgas und CO₂ unter Wärmezufuhr und in Abwesenheit von Wasser zu Synthesegas mit einem stöchiometrischen Verhältnis von H₂ und CO von etwa 1:1. Die Trockenreformierung im Sinne der Erfindung umfasst insbesondere auch die Umsetzung von CH₄ oder Erdgas und CO₂ in Anwesenheit von Wasserdampf, wobei Wasser nur in einem stöchiometrischen Verhältnis zu Methan oder Erdgas von 1:2, 1:3, 1:4, 1:5, 1:10 oder 1:20 anwesend ist. Generell wird im Rahmen dieser Erfindung insbesondere von einer Trockenrefomierung gesprochen, wenn das molare Verhältnis von Wasser zu Kohlenstoff im Einsatz kleiner als 2:1, bevorzugt kleiner als 1:1, ist.

Weiterhin kann DME in einem Olefinsyntheseschritt zu einem Produkt umfassend Olefine, insbesondere Ethylen und/oder Propylen, umgesetzt werden, wobei der Produktstrom direkt dem Olefinsyntheseschritt zugeführt wird. Alternativ kann lediglich CO₂ aus dem Produktstrom abgetrennt werden und sodann der Produktstrom dem Olefinsyntheseschritt zugeführt werden.

Mit besonderen Vorteilen wird die Direktsynthese im DME-Reaktor und/oder die Erzeugung des Synthesegases bei einem Druck von 20 bar bis 70 bar, vorzugsweise von 30 bis 50 bar durchgeführt wird. Insbesondere ist es von Vorteil, wenn Direktsynthese im DME-Reaktor und die Erzeugung des Synthesegases beim gleichen Druck oder bei einem Druck, der um höchsten 3 bar, vorzugsweise höchsten 1 bar, voneinander abweicht, vorgenommen wird. Die Druckdifferenz der Direktsynthese im DME-Reaktor und der Erzeugung des Synthesegases beträgt dabei am Ausgang der Erzeugung des Synthesegases und am Eingang in den DME-Reaktor höchstens 3 bar, vorzugsweise höchsten 1 bar.

Weitere Einzelheiten und Vorteile der Erfindung sollen durch die nachfolgenden Figurenbeschreibungen von Ausführungsbeispielen anhand der Figuren erläutert werden.

Es zeigt:

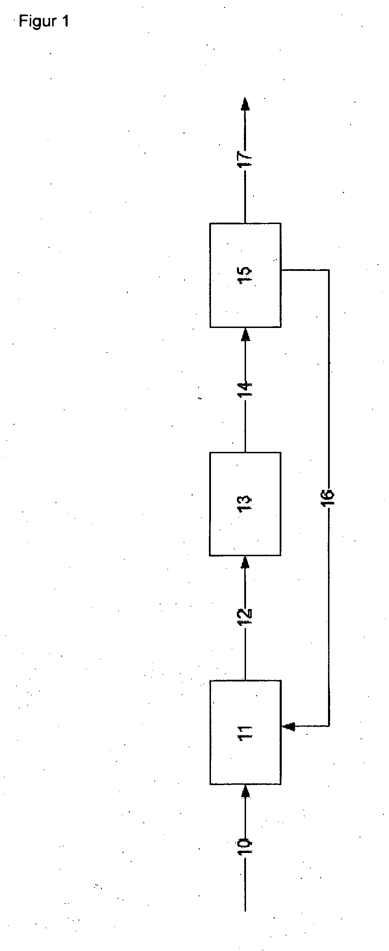

- Fig. 1: ein Blockdiagramm eines erfindungsgemäßen Verfahrens.

Figur 1 zeigt ein Blockdiagramm eines erfindungsgemäßen Verfahrens zur Herstellung von DME mittels Direktsynthese aus Synthesegas (CO und H₂).

Hierbei wird Synthesegas in einem Synthesegasschritt (Synthesegaserzeugung) 11 aus Erdgas 10 erzeugt, beispielsweise durch Dampfreformierung. Das Synthesegas 12 wird in einen DME Reaktor eingeleitet und in Anwesenheit eines entsprechend ausgelegten Cu-basierten Katalysators (siehe oben) zu DME umgesetzt, wobei der Reaktor nahe am bzw. am chemischen Gleichgewicht betrieben wird:

3H₂ + 3CO -> DME + CO₂.

Hierduch wird vergleichweise wenig Synthesegas nicht umgesetzt, wodurch die nachfolgenden Trennaufgaben erheblich erleichtert werden, da nunmehr das Synthesegas nicht mehr vom CO₂ abgetrennt werden muss.

Der Reaktoreffluentstrom (Produktstrom) 14 enthaltend CO, H₂, CO₂, DME, H₂O und Methanol wird aus dem DME-Reaktor 13 einem Trennschritt 15 zugeführt, in dem das Reaktoreffluent gekühlt wird, so dass eine gasförmige Phase enthaltend CO, H₂ und CO₂ erhalten wird, die in den Syntheseteil 14 zurückgeführt wird sowie eine flüssige Phase 17, in der DME angereichert ist.

**Bezugszeichenliste:**

| | |
|---|---|
| 10 | Erdgas |
| 11 | Synthesegaserzeugung |
| 12 | Synthesegas (CO + H₂) |
| 13 | DME-Reaktor für Direktsynthese |
| 14 | Reaktoreffluent (Produktstrom) |
| 15 | Kühlung, Gas-Flüssigkeit-Trennung |
| 16 | Gasförmige Phase (CO₂, CO, H₂) |
| 17 | Flüssige Phase mit DME |

## Patentansprüche

1. Verfahren zur Herstellung von DME (17), aufweisend die Schritte:
- Erzeugen eines Synthesegases (12) enthaltend CO und H₂ in einer Einrichtung (11) zur Synthesegaserzeugung,
- Einleiten des Synthesegases (12) in einen DME-Reaktor (13) zur Direktsynthese von DME durch katalysierte Umsetzung des Synthesegases (12) mittels eines Katalysators unter Bildung eines Produktstromes (14) enthaltend DME, CO₂, H₂O, CH₃OH und nicht umgesetztes Synthesegas (12), wobei die Direktsynthese im DME-Reaktor (13) zumindest bis zum Erreichen einer Konzentration des DME im Produktstrom (14) von 70%, 80%, 85% oder 90% der chemischen Gleichgewichtskonzentration von DME durchgeführt wird, und wobei
- der Produktstrom (14) enthaltend CO, H₂, CO₂, DME, H₂O und Methanol zur Erzeugung einer gasförmigen Phase (16) enthaltend CO₂, H₂ und CO sowie einer flüssigen Phase (17) enthaltend DME gekühlt wird, und wobei
- die gasförmige Phase (16) in die Einrichtung (11) zur Synthesegaserzeugung zurückgeführt wird.

2. Verfahren nach Anspruch 1, **dadurch gekennzeichnet, dass** der Katalysator ein Cu-basierter Katalysator mit einer aciden Funktionalität ist, die insbesondere die Wasserabspaltung gemäß 2CH₃OH -> DME + H₂O begünstigt.

3. Verfahren nach einem der vorhergehenden Ansprüche, **dadurch gekennzeichnet, dass** das Synthesegas (12) durch Trockenreformierung erzeugt wird, wobei Methan und Kohlendioxid zu Kohlenmonoxid und Wasserstoff umgesetzt werden.

4. Verfahren nach einem der vorhergehenden Ansprüche, **dadurch gekennzeichnet, dass** DME in einem Olefinsyntheseschritt zu einem Produkt umfassend Olefine, insbesondere Ethylen und/oder Propylen, umgesetzt wird, wobei der Produktstrom direkt dem Olefinsyntheseschritt zugeführt wird, oder dass lediglich CO₂ aus dem Produktstrom abgetrennt wird und sodann der Produktstrom dem Olefinsyntheseschritt zugeführt wird.

5. Verfahren zur Herstellung von DME (17), aufweisend die Schritte:
- Erzeugen eines Synthesegases (12) enthaltend CO und H₂ in einer Einrichtung (11) zur Synthesegaserzeugung,
- Einleiten des Synthesegases (12) in einen DME-Reaktor (13) zur Direktsynthese von DME durch katalysierte Umsetzung des Synthesegases (12) mittels eines Katalysators unter Bildung eines Produktstromes (14) enthaltend DME, CO₂, H₂O, CH₃OH und nicht umgesetztes Synthesegas (12),
- wobei der Katalysator dazu ausgebildet ist, die Wasserabspaltung gemäß 2CH₃OH->DME+H₂O zu beschleunigen, und
- wobei in der Direktsynthese gebildetes Kohlendioxid (16) und nicht umgesetztes Synthesegas (12) zur Synthesegaserzeugung zurückgeleitet wird.

6. Verfahren nach Anspruch 5, **dadurch gekennzeichnet, dass** der Katalysator ein Cu-basierter Katalysator mit einer aciden Funktionalität ist.

7. Verfahren nach Anspruch 5 oder 6, **dadurch gekennzeichnet, dass** das Synthesegas (12) durch Trockenreformierung erzeugt wird, wobei Methan und Kohlendioxid zu Kohlenmonoxid und Wasserstoff umgesetzt werden.

8. Verfahren nach einem der Ansprüche 5 bis 7, **dadurch gekennzeichnet, dass** DME in einem Olefinsyntheseschritt zu einem Produkt umfassend Olefine, insbesondere Ethylen und/oder Propylen, umgesetzt wird, wobei der Produktstrom direkt dem Olefinsyntheseschritt zugeführt wird, oder dass lediglich CO₂ aus dem Produktstrom abgetrennt wird und sodann der Produktstrom dem Olefinsyntheseschritt zugeführt wird.

9. Verfahren nach einem der Ansprüche 1 bis 8, **dadurch gekennzeichnet, dass** die Direktsynthese im DME-Reaktor (13) und/oder die Erzeugung des Synthesegases (12) bei einem Druck von 20 bar bis 70 bar, vorzugsweise von 30 bis 50 bar durchgeführt wird.

10. Verfahren nach einem der Ansprüche 1 bis 9, **dadurch gekennzeichnet, dass** die Direktsynthese im DME-Reaktor (13) bei einer Temperatur zwischen 150 °C und 400 °C, vorzugsweise zwischen 200 °C und 320 °C durchgeführt wird.

## Claims

1. Process for production of DME (17) comprising the steps of:
- generating a syngas (12) containing CO and H₂ in a device (11) for syngas generation,
- passing the syngas (12) into a DME reactor (13) for direct synthesis of DME by catalysed conversion of the syngas (12) by means of a catalyst to form a product stream (14) containing DME, CO₂, H₂O, CH₃OH and unconverted syngas (12), wherein the direct synthesis in the DME reactor (13) is at least carried on until achievement of a DME concentration in product stream (14) amounting to 70%, 80%, 85% or 90% of the chemical equilibrium concentration of DME, and wherein
- the product stream (14) containing CO, H₂, CO₂, DME, H₂O and methanol is cooled to generate a gaseous phase (16) containing CO₂, H₂ and CO and a liquid phase (17) containing DME, and wherein
- the gaseous phase (16) is recycled into the device (11) for syngas generation.

2. Process according to Claim 1, **characterized in that** the catalyst is a Cu-based catalyst having an acidic functionality which favours in particular the elimination of water as per 2CH₃OH -> DME + H₂O.

3. Process according to either preceding claim, **characterized in that** the syngas (12) is generated by dry reforming wherein methane and carbon dioxide are converted into carbon monoxide and hydrogen.

4. Process according to any preceding claim, **characterized in that** DME is converted in an olefin synthesis step into a product comprising olefins, especially ethylene and/or propylene, wherein the product stream is fed directly to the olefin synthesis step, or **in that** merely CO₂ is separated from the product stream and then the product stream is fed to the olefin synthesis step.

5. Process for production of DME (17) comprising the steps of:
- generating a syngas (12) containing CO and H₂ in a device (11) for syngas generation,
- passing the syngas (12) into a DME reactor (13) for direct synthesis of DME by catalysed conversion of the syngas (12) by means of a catalyst to form a product stream (14) containing DME, CO₂, H₂O, CH₃OH and unconverted syngas (12),
- wherein the catalyst is designed to hasten the elimination of water as per 2CH₃OH->DME+H₂O, and
- wherein carbon dioxide (16) formed in the direct synthesis and/or syngas (12) not converted in the direct synthesis is returned for syngas generation.

6. Process according to Claim 5, **characterized in that** the catalyst is a Cu-based catalyst having an acidic functionality.

7. Process according to Claim 5 or 6, **characterized in that** the syngas (12) is generated by dry reforming wherein methane and carbon dioxide are converted into carbon monoxide and hydrogen.

8. Process according to any of Claims 5 to 7, **characterized in that** DME is converted in an olefin synthesis step into a product comprising olefins, especially ethylene and/or propylene, wherein the product stream is fed directly to the olefin synthesis step, or **in that** merely CO₂ is separated from the product stream and then the product stream is fed to the olefin synthesis step.

9. Process according to any of Claims 1 to 8, **characterized in that** the direct synthesis in the DME reactor (13) and/or the generation of syngas (12) is carried out at a pressure of 20 bar to 70 bar, preferably of 30 to 50 bar.

10. Process according to any of Claims 1 to 9, **characterized in that** the direct synthesis in the DME reactor (13) is carried out at a temperature between 150°C and 400°C, preferably between 200°C and 320°C.

## Revendications

1. Procédé de fabrication de DME (17), comprenant les étapes suivantes :
- la formation d'un gaz de synthèse (12) contenant du CO et de l'H₂ dans un dispositif (11) pour la formation d'un gaz de synthèse,
- l'introduction du gaz de synthèse (12) dans un réacteur de DME (13) pour la synthèse directe de DME par réaction catalysée du gaz de synthèse (12) au moyen d'un catalyseur avec formation d'un courant de produits (14) contenant du DME, du CO₂, de l'H₂O, du CH₃OH et du gaz de synthèse non réagi (12), la synthèse directe dans le réacteur de DME (13) étant réalisée au moins jusqu'à atteindre une concentration du DME dans le courant de produits (14) de 70 %, 80 %, 85 % ou 90 % de la concentration à l'équilibre chimique de DME, et
- le courant de produits (14) contenant du CO, de l'H₂, du CO₂, du DME, de l'H₂O et du méthanol étant refroidi pour former une phase gazeuse (16) contenant du CO₂, de l'H₂ et du CO, et une phase liquide (17) contenant du DME, et
- la phase gazeuse (16) étant recyclée dans le dispositif (11) pour la formation du gaz de synthèse.

2. Procédé selon la revendication 1, **caractérisé en ce que** le catalyseur est un catalyseur à base de Cu ayant une fonctionnalité acide, qui favorise notamment le clivage de l'eau selon 2 CH₃OH -> DME + H₂O.

3. Procédé selon l'une quelconque des revendications précédentes, **caractérisé en ce que** le gaz de synthèse (12) est formé par reformage à sec, du méthane et du dioxyde de carbone étant transformés en monoxyde de carbone et hydrogène.

4. Procédé selon l'une quelconque des revendications précédentes, **caractérisé en ce que** le DME est transformé dans une étape de synthèse d'oléfines en un produit comprenant des oléfines, notamment de l'éthylène et/ou du propylène, le courant de produits étant introduit directement dans l'étape de synthèse d'oléfines, ou **en ce qu'**uniquement le CO₂ est séparé du courant de produits, puis le courant de produits est introduit dans l'étape de synthèse d'oléfines.

5. Procédé de fabrication de DME (17), comprenant les étapes suivantes :
- la formation d'un gaz de synthèse (12) contenant du CO et de l'H₂ dans un dispositif (11) pour la formation d'un gaz de synthèse,
- l'introduction du gaz de synthèse (12) dans un réacteur de DME (13) pour la synthèse directe de DME par réaction catalysée du gaz de synthèse (12) au moyen d'un catalyseur avec formation d'un courant de produits (14) contenant du DME, du CO₂, de l'H₂O, du CH₃OH et du gaz de synthèse non réagi (12),
- le catalyseur étant configuré de manière à accélérer le clivage de l'eau selon 2CH₃OH -> DME + H₂O, et
- le dioxyde de carbone (16) formé lors de la synthèse directe et le gaz de synthèse non réagi (12) étant réintroduits dans la formation du gaz de synthèse.

6. Procédé selon la revendication 5, **caractérisé en ce que** le catalyseur est un catalyseur à base de Cu ayant une fonctionnalité acide.

7. Procédé selon la revendication 5 ou 6, **caractérisé en ce que** le gaz de synthèse (12) est formé par reformage à sec, du méthane et du dioxyde de carbone étant transformés en monoxyde de carbone et hydrogène.

8. Procédé selon l'une quelconque des revendications 5 à 7, **caractérisé en ce que** le DME est transformé dans une étape de synthèse d'oléfines en un produit comprenant des oléfines, notamment de l'éthylène et/ou du propylène, le courant de produits étant introduit directement dans l'étape de synthèse d'oléfines, ou **en ce qu'**uniquement le CO₂ est séparé du courant de produits, puis le courant de produits est introduit dans l'étape de synthèse d'oléfines.

9. Procédé selon l'une quelconque des revendications 1 à 8, **caractérisé en ce que** la synthèse directe dans le réacteur de DME (13) et/ou la formation du gaz de synthèse (12) sont réalisées à une pression de 20 bar à 70 bar, de préférence de 30 à 50 bar.

10. Procédé selon l'une quelconque des revendications 1 à 9, **caractérisé en ce que** la synthèse directe dans le réacteur de DME (13) est réalisée à une température comprise entre 150 °C et 400 °C, de préférence entre 200 °C et 320 °C.
